# EUROPEAN PATENT SPECIFICATION

(11) **EP 2 925 331 B1**
(45) Date of publication and mention of the grant of the patent: **31.01.2018**
(21) Application number: 12889340.1
(22) Date of filing: 28.11.2012
(51) Int. Cl.: A61K 33/18, A61K 33/44, A61P 11/00

(54) **ACTIVATED CARBON COMPRISING AN ADSORBED IODIDE SALT IN A METHOD FOR TREATING CHRONIC BRONCHITIS**
AKTIVKOHLE MIT EINEM ADSORBIERTEN JODIDSALZ IN EINEM VERFAHREN ZUR BEHANDLUNG VON CHRONISCHER BRONCHITIS
CHARBON ACTIF COMPRENANT UN SEL D'IODURE ADSORBÉ DANS UN PROCÉDÉ DE TRAITEMENT DE LA BRONCHITE CHRONIQUE

(43) Date of publication of application: 07.10.2015
(73) Proprietor: Pharmalundensis AB, 223 81 Lund (SE)
(72) Inventor: SKOGVALL, Staffan, 224 72 Lund (SE)
(74) Representative: Awapatent AB
(86) International application number: PCT/SE2012/051313
(87) International publication number: WO 2014/084763

(56) References cited:
- WO-A1-2009/067067
- WO-A1-2009/067067
- WO-A1-2009/078782
- WO-A1-2009/078782
- WO-A2-2007/112248
- US-A1- 2002 099 023

## Description

The present invention relates to treatment of chronic bronchitis. In particular, the present invention aims at providing use of activated carbon comprising an adsorbed iodide salt in a method for treating increased sputum production and cough caused by chronic bronchitis.

### Technical background

Chronic bronchitis is characterized by cough and increased sputum production for at least three months per year in two consecutive years. If bronchitis appears together with emphysema it is called chronic obstructive pulmonary disease (COPD). Chronic bronchitis was recently shown to have a prevalence of ∼5-6 % (Pahwa et al., J Occup Environ Med. 2012 Oct 30.

### [Epub ahead of print]).

Chronic bronchitis is primarily caused by cigarette smoking, second hand smoke, and air pollution, although other factors may be of importance as well. The main goals in the treatment of chronic bronchitis is to keep the airways open and functioning properly, to help clear the airways of mucus to avoid lung infections and to prevent further disability. In spite of this, chronic bronchitis often progresses to COPD, which is the 4^{th} most common cause of death in the western world.

It has previously been suggested that airway obstruction caused by chronic obstructive pulmonary disease, COPD, can be reduced by administration of the mercury binding conjugate "iodinated activated charcoal" (WO 2009/067067) even though nothing is disclosed about treating the milder condition chronic bronchitis. However, one drawback with administering activated charcoal impregnated with iodine is that it contains high amounts of iodine, which may be harmful to humans. Another problem with using iodinated activated charcoal as a medication is that elemental iodine is highly reactive and the conjugate can therefore not be formulated in a standard capsule or tablet.

Accordingly, there is a need for an improved preparation for treating chronic bronchitis.

### Summary of the invention

It has now turned out that an improved preparation for treating chronic bronchitis can be obtained by the subject matter of present claim 1. Accordingly, in a first aspect, the present invention provides activated carbon comprising an adsorbed iodide salt selected from the group of alkali metal iodides and earth alkali iodides, for use in a method for treating chronic bronchitis.

As disclosed herein, the term "activated carbon" also includes "activated charcoal".

Typical examples of such iodides that could be used in the present invention are NaI, KI, MgI₂, and CaI₂. Preferably, KI is included as adsorbed such iodide.

Preferably, the amount adsorbed iodide salt is within the range of 0.25 - 10 % (wt.), and preferably within the range of 0.5 - 5 % (wt.).

Preferably, the activated carbon also comprises an adsorbed pharmaceutically acceptable bromide salt, such as sodium bromide, potassium bromide, magnesium bromide, lithium bromide, ammonium bromide and/or calcium bromide. The amount of bromide salt may be within the range of 1 - 1000 % (wt.) calculated on the weight of the adsorbed iodide salt.

### Detailed description of preferred embodiments

In an attempt to solve the problems mentioned in the technical background section above, it was examined if it would be possible to impregnate activated charcoal with something which retains the mercury-binding capacity of the activated charcoal, but which is less potentially harmful to humans and which is less reactive. It was surprisingly found that impregnation with potassium iodide resulted in a much higher specific mercury-binding capacity compared to impregnation with iodine. As a matter of fact, activated charcoal impregnated with 1.6 % KI was found to bind as much mercury as activated charcoal impregnated with 8 % iodine. In addition, potassium iodide-impregnated activated charcoal may, in contrast to I₂-impregnated activated charcoal, be placed in standard capsules without risk of any undesired side reactions between the capsule material and the active component.

Typically, the activated charcoal impregnated with an iodide salt is administrated to a human or animal in need thereof in a pharmaceutical composition comprising said impregnated activated charcoal together with a pharmaceutically acceptable excipient. The selection of excipient is not critical and most commonly used acceptable excipients could be included in such a pharmaceutical composition.

Preferably, the pharmaceutical composition is selected from the group of an aqueous suspension, a capsule, a powder for preparing an oral suspension or a tablet. For instance, when the activated carbon comprising adsorbed iodide salt is administered in the form of a tablet or capsule, said activated carbon comprising adsorbed iodide salt can be combined with an oral, non-toxic pharmaceutically acceptable inert carrier such as ethanol, glycerol, water and the like. Moreover, when desired or necessary, suitable binders, lubricants, disintegrating agents and colouring agents can also be incorporated into the mixture. Suitable binders include, without limitation, starch, gelatine, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

It is preferred that the pharmaceutical composition also comprises a bile secretion stimulating agent. Typically, said bile secretion stimulating agent is fat.

The pharmaceutical composition should preferably be administered in the interval between two meals, such as one or two or three hours after a meal and one or two or three or more hours prior to a meal. The composition could also be administered before breakfast.

In some embodiments, particulate activated charcoal loaded with adsorbed alkali metal or earth alkali metal iodide can be administered filled in soft or hard gelatine capsules, vegetable or pullulan capsules, or in form of a tablet formed from particulate activated charcoal loaded with adsorbed alkali metal iodide or earth alkali metal iodide and a suitable pharmaceutically acceptable binder. The binder should be of a kind allowing the tablet to disintegrate in gastrointestinal fluid. Suitable binders comprise chemically modified cellulose, such as carboxymethyl cellulose and polyvinyl pyrrolidone. Because of the fragile nature of the carbon, only slight compression should be used when forming the tablets so as not to crush the carbon particles.

Preferably, the pharmaceutically effective amount of activated carbon loaded with an adsorbed alkali metal or earth alkali metal iodide is administered daily and may include 1 - 3 daily administrations. However, the administration may also be intermittent and involve administration every second or third day or once or more per week.

### Experimental work:

### EXAMPLE 1: Preparation of activated carbon samples loaded alkali metal iodide or earth alkali metal iodide.

### Materials:

Activated carbon (Sigma C7606), Potassium iodide (Sigma P7744), deionized water.

### Equipment:

Magnetic stirrer IKA RTC basic, oil bath, reflux condenser, balance XP-300 (Denver instruments), Pyrex glass flask (2 L), polymer-enclosed magnetic bar, vacuum filter flask (2 L), OOH filter paper (Whatman), laboratory drying oven TS80000, Termaks.

### Method:

8.0 g KI was dissolved in 1 L of water in an adsorption experiment. Activated carbon (92 g) was added. The suspension was stirred for 12 hours at room temperature (21 - 23°C). The activated carbon product was separated from the KI solution by filtration under reduced pressure and dried for 12 h in 75°C. This resulted in a sample consisting of activated carbon impregnated with ∼1.6 % (wt.) KI. Activated carbon preparations coated with other specific amounts of KI were obtained by repeating example 1 using other amounts of activated carbon and KI. Determination of the amount of adsorbed KI on activated carbon was carried out by three methods, namely conductometry, gravimetric analysis and elemental analysis.

### EXAMPLE 2: Analysis of mercury uptake in activated carbon samples impregnated with KI

### Materials:

KI-impregnated carbon sample. Trizma, Sigma T1503-100G. Sodium chloride: Sigma, S988-500G. Potassium Chloride: Fluka 60130-1000G. Deionized water. Hydrochloric acid: Sigma 84422-1L. Mercury (II) chloride 99.5 % min., Alfa Aesar. Nitric acid: Sigma 30702, min 69 %, puriss.

### Equipment:

Water bath with stirrer and thermostat RCT B, IKA, Germany. Magnetic stirring bar. Volumetric glass flask, 500 ml. Round bottom glass flask, 1000 ml, with stopper, Safety pipette, 25 ml. Filter paper 00H grade, diameter 150 mm, Whatman.

### Preparation of saline solution:

A buffer solution was prepared, containing 0.01 M Trizma, 140 mM NaCl and 4 mM KCI, and adjusted to pH 7.4 with HCl. The buffer solution was capped, preheated and kept at 37 °C.

### Preparation of mercury (II) chloride stock solution (approx. 10⁻³ M):

About 0.027 g HgCl₂ was weighed on an analytical balance. The exact weight was noted. The HgCl₂ was transferred to the 100 ml volumetric flask and diluted with de-ionized water until it had a concentration of 10⁻³ M.

### Preparation of test solution with mercury (II) chloride (10⁻⁵ M):

5 ml of HgCl₂ stock solution was added to the 500 ml measuring flask using the automatic pipette. Preheated 37 °C buffer solution was added up to the 500 ml mark. The test solution was transferred to the 1000 ml round bottom flask. The flask was closed with a stopper and placed in the water bath at 37°C.

Binding of mercury from the test solution to activated carbon loaded with KI: 50 mg of the activated carbon loaded with KI is added to the test solution containing 10⁻⁵ M mercury (II) chloride, and absorption of mercury in the impregnated carbon was allowed to proceed for 30 min while stirring at 300 rpm. When the absorption is complete, a 20 ml sample was withdrawn with the safety pipette and filtered. The sample was added to an amber bottle containing 2 % HNO₃ and analyzed.

### Analysis:

The sample was analyzed by atomic fluorescence spectrometry. The analytical result of mercury (Hg) was reported as mg/L.

### Results:

In this model, activated carbon impregnated with 1.6 % (wt.) of KI was found to bind 98 % of available HgCl₂.

### EXAMPLE 3 (reference). Preparation of iodinated activated carbon

### Materials:

Activated carbon from Sigma C7606; meets USP testing specification. Elemental Iodine from Sigma-Aldrich 03002; meets USP testing. Undenatured ethanol from Kemetyl; meets USP and EP testing specifications with < 0.5 % water content.

### Equipment:

Mixing cylinder 500 ml, measuring cylinder 500 ml, E-flask 50 ml, Büchner funnel Duran diameter 105 ml and stirrer motor with blade, RZR 1 from Heidolph. Filterpaper grade 00H from Munktell. Evaporation dish made from borosilicate glass.

Method: Depending on the batch size, the amount of activated carbon, elemental iodine and ethanol is calculated. For a batch size of 50 g iodinated carbon, 4.5 g of iodine, 45.5 g of activated carbon and 450 ml ethanol is used. The activated carbon is suspended in the measuring cylinder with 410 ml ethanol and the elemental iodine is solved in the E-flask with 40 ml ethanol. The iodine is added, stirred for 2 min and allowed to impregnate the carbon for 1 h. Thereafter, the iodinated activated carbon is separated from the ethanol solution by filtration under reduced pressure and dried for 5 hours at 150 °C. This results in iodinated activated carbon impregnated with 9 % (wt.) I₂. The amount of adsorbed iodine is determined by elemental analysis.

### EXAMPLE 4. Further analysis of mercury uptake in activated carbon comprising adsorbed potassium iodide or iodine (activated carbon comprising adsorbed iodine serves as comparison)

The experiment of Example 2 was repeated but the amount of potassium iodide adsorbed on the activated carbon was varied. Similar to example 2, mercury was present as HgCl₂ dissolved in de-ionized water. The amount of remaining dissolved mercury was determined by atomic fluorescence in the same way as in Example 2. Furthermore, the results were compared with the results obtained for similar amounts of iodine adsorbed on activated carbon. Preparation of activated carbon samples loaded with different amounts of potassium iodide was performed according to example 1. Activated carbon comprising adsorbed iodine was prepared using the method of example 3 although the amounts of iodine were varied.

The results obtained are provided below:

| **Activated charcoal impregnated with KI** | | | |
|---|---|---|---|
| KI in impregnation solution (%) | Adsorbed KI on activated carbon (% (wt.)) | Remaining amount of mercury in solution (mg/l) | Remaining amount of mercury in solution (% (wt.)) |
| Control mercury concentration before charcoal | | 2.0 | 100 |
| Activated carbon with adsorbed KI (%) | | | |
| 0 | 0 | 0.66 | 33 |
| 1.0 | 0.55 | 0.34 | 17 |
| 3.0 | 1.09 | 0.14 | 7 |
| 8.0 | 1.62 | 0.047 | 2 |

These experiments were repeated with iodine instead of KI. The following results were obtained:

| **Activated charcoal impregnated with I₂** | | | |
|---|---|---|---|
| I₂ in impregnation solution (%) | Adsorbed I₂ on activated carbon (% (wt.) | Remaining amount of mercury in solution (mg/l) | Remaining amount of mercury in solution (% (wt.)) |
| Control mercury concentration before charcoal | | 20 | 100 |
| Activated carbon with adsorbed I₂ (%) | | | |
| 0 | 0 | 8 | 40 |
| 1 | 1 | 7.8 | 39 |
| 3 | 3 | 5.3 | 26 |
| 8 | 8 | 0.78 | 3.9 |

The following conclusions were drawn based on the obtained results:
All of the I₂ dissolved in the impregnation solution (even when said solution contained 8 % (wt.)) was adsorbed to the activated charcoal. When activated carbon was exposed to a solution containing 8 % (wt.) KI, surprisingly only 1.62 % (wt.) was adsorbed on the activated charcoal. This small amount of KI does, however, increase the mercury-binding capacity of activated charcoal by more than 10 times, which is equal to the mercury-binding capacity obtained when activated charcoal is impregnated with 8 % I₂. This is unexpected and very advantageous because iodine may be harmful to humans and animals in high amounts and also because KI is less reactive than iodine. Substitution of potassium iodide for iodine leads to substantial reduction of risks for toxic side-effects in humans and animals combined with reduced risks for undesired reactions with components in the pharmacological administration form used, such as capsules or tablets.

### EXAMPLE 5: Use of KI-impregnated charcoal for treating a patient with chronic bronchitis

A 60 year old Caucasian male has for several years experienced increasing problems with cough and mucous production for long periods. He was diagnosed as suffering from chronic bronchitis. He was prescribed standard treatment including corticosteroids, anti-cholinergics, beta2-stimulants and mucolytics such as acetylcysteine. This, however, did not reduce the cough or mucous production substantially.

Since the chronic bronchitis caused considerable inconvenience, he examined alternative ways to reduce the symptoms. When taking 300 mg/day of activated charcoal impregnated with 1.6 % KI in a 00 vegetable capsule for one month, both the cough and the sputum production were reduced considerably.

In order to clarify if the improvement was caused by activated charcoal, he took 300 mg activated charcoal without iodide for one month. However, this resulted in a worsening of the cough and sputum production back to the original situation.

To clarify if the improvement of the chronic bronchitis by KI-impregnated activated charcoal was caused by iodide, he then took a capsule with 10 mg (which amount is considerably higher than the amount of iodide in the KI-impregnated activated charcoal, 300 mg x 1.62 % = 4.9 mg) KI per day for one month. This did not reduce the cough or sputum production at all.

Finally, he once again took 300 mg activated charcoal impregnated with 1.6 % KI in a 00 vegetable capsule for one month. Once again, the cough and the sputum production were reduced considerably.

Potassium iodide has previously been used to treat chronic obstructive pulmonary disease (Bernecker, C. Intermittent therapy with potassium iodide in chronic obstructive disease of the airways. Acta Allergologica, 1969, 216-225). However, much higher amounts were given (1.5 - 3 g and more). Ammoniated potassium iodide mixture (150 - 300 mg per dose) was also used. These are much higher amounts than the ∼5 mg/day used in the above example. Also, when capsules with only KI was used in the above example, no improvement of cough or sputum production was found. Thus, it appears essential that both KI and activated charcoal are administered simultaneously in order to improve the chronic bronchitis.

## Claims

1. Activated carbon comprising an adsorbed iodide salt selected from the group of alkali metal iodides and earth alkali iodides, for use in a method for treating chronic bronchitis.

2. Activated carbon for use according to claim 1, **characterized in that** the amount of adsorbed iodide salt is within the range of 0.25 -10 % (wt), and preferably within the range of 0.5-5 % (wt).

3. Activated carbon for use according to any of claims 1 and 2, **characterized in that** the adsorbed iodide is potassium iodide.

4. Activated carbon for use according to any of claims 1 -3, **characterized in that** the activated carbon also comprises a pharmaceutically acceptable bromide salt, such as sodium bromide, potassium bromide, lithium bromide, ammonium bromide and/or calcium bromide.

## Patentansprüche

1. Aktivkohle, die ein adsorbiertes Jodidsalz ausgewählt aus der Gruppe von Alkalimetall-Jodiden und Erdalkali-Jodiden umfasst, zur Verwendung in einem Verfahren zur Behandlung von chronischer Bronchitis.

2. Aktivkohle zur Verwendung nach Anspruch 1, **dadurch gekennzeichnet, dass** die Menge von adsorbiertem Jodidsalz innerhalb des Bereiches von 0,25 - 10 % (Gew.) und vorzugsweise innerhalb des Bereiches von 0,5 - 5 % (Gew.) liegt.

3. Aktivkohle zur Verwendung nach einem der Ansprüche 1 und 2, **dadurch gekennzeichnet, dass** das adsorbierte Jodid Kaliumjodid ist.

4. Aktivkohle zur Verwendung nach einem der Ansprüche 1 - 3, **dadurch gekennzeichnet, dass** die Aktivkohle auch ein pharmazeutisch akzeptables Bromidsalz, wie z.B. Natriumbromid, Kaliumbromid, Lithiumbromid, Ammoniumbromid und/oder Calciumbromid, umfasst.

## Revendications

1. Charbon actif comprenant un sel d'iodure adsorbé sélectionné dans le groupe des iodures de métaux alcalins et des iodures de métaux alcalino-terreux, pour son utilisation dans un procédé de traitement de la bronchite chronique.

2. Charbon actif pour son utilisation selon la revendication 1, **caractérisé en ce que** la quantité de sel d'iodure adsorbé se trouve dans la plage de 0,25 - 10 % (p), et de préférence dans la plage de 0,5 - 5 % (p).

3. Charbon actif pour son utilisation selon l'une quelconque des revendications 1 et 2, **caractérisé en ce que** l'iodure adsorbée est l'iodure de potassium.

4. Charbon actif pour son utilisation selon l'une quelconque des revendications 1 - 3, **caractérisé en ce que** le charbon actif comprend également une quantité pharmaceutiquement acceptable de sel de bromure, par exemple de bromure de sodium, de bromure de potassium, de bromure de lithium, de bromure d'ammonium et/ou de bromure de calcium.
